# EUROPEAN PATENT SPECIFICATION

(11) **EP 4 241 808 B1**
(45) Date of publication and mention of the grant of the patent: **25.02.2026**
(21) Application number: 22185067.0
(22) Date of filing: 15.07.2022
(51) Int. Cl.: A61M 5/315, A61M 5/142, A61M 5/20

(54) **DRIVING MECHANISM FOR DRIVING A PLUNGER OF AN AUTO-INJECTOR TO SLIDE RELATIVE TO A RESERVOIR OF THE AUTO-INJECTOR AND AUTO-INJECTOR THEREWITH**
ANTRIEBSMECHANISMUS ZUM ANTREIBEN EINES KOLBENS EINES AUTO-INJEKTORS ZUM GLEITEN RELATIV ZU EINEM RESERVOIR DES AUTO-INJEKTORS UND DES AUTO-INJEKTORS DAMIT
MÉCANISME D'ENTRAÎNEMENT D'UN PISTON D'UN AUTO-INJECTEUR POUR LE FAIRE GLISSER PAR RAPPORT AU RÉSERVOIR DE L'AUTO-INJECTEUR ET AUTO-INJECTEUR ASSOCIÉ

(30) Priority: 11.03.2022 US 202263318785 P; 19.06.2022 US 202217844038
(43) Date of publication of application: 13.09.2023
(73) Proprietor: Altek Biotechnology Corporation, 300 Hsinchu City (TW)
(72) Inventor: Huang, Yu-Cheng, 300 Hsinchu City (TW)
(74) Representative: Straus, Alexander

(56) References cited:
- EP-A1- 3 442 623
- EP-B1- 3 442 623
- WO-A1-2020/160821
- DE-T5- 112016 004 690
- US-A1- 2005 090 781
- US-A1- 2018 126 068
- US-B1- 6 179 569
- US-B2- 9 308 324

## Description

### Field of the Invention

The present invention relates to a driving mechanism and an auto-injector therewith according to the pre-characterizing clauses of claims 1 and 10.

### Background of the Invention

An auto-injector, e.g., an on-body injector, is a medical device designed to deliver a dose of a drug. However, the conventional auto-injectors available in the markets are unable to meet requirements of small volume, high driving power, long driving distance, long injecting period and accurate drug dose delivery rate. For example, in EP 3442623 A1, it discloses a syringe for delivering microliter-sized doses, but the syringe has complicated structure and is not suitable for high driving power due to poor structural strength. Furthermore, in WO 2020160821 A1, it discloses an infusion device for administering a medical fluid to a patient, but the infusion device has large size and fails to provide accurate drug dose delivery rate. Besides, in US 9308324 B2, it discloses a medicine injection device for dispensing a medicine, but the medicine injection device also has complicated structure and fails to provide accurate drug dose delivery rate. Therefore, an improvement of the auto-injector is urgently needed.

### Summary of the Invention

This in mind, the present invention aims at providing a driving mechanism for driving a plunger of an auto-injector to slide relative to a reservoir of the auto-injector, which can meet requirements of small volume, high driving power, long driving distance, long injecting period and accurate drug dose delivery rate, and an auto-injector therewith.

This is achieved by a driving mechanism and an auto-injector therewith according to claims 1 and 10. The dependent claims pertain to corresponding further developments and improvements.

As will be seen more clearly from the detailed description following below, the claimed driving mechanism is for driving a plunger of an auto-injector to slide relative to a reservoir of the auto-injector. The driving mechanism includes a first transmission component, a driving component, a second transmission component, a third transmission component, a sliding component and a bracket. The driving component is coupled to the first transmission component and for driving the first transmission component to rotate. The second transmission component is rotatably engaged with the first transmission component. The second transmission component is driven by the first transmission component to rotate when the driving component drives the first transmission component to rotate. The third transmission component is fixedly connected to the second transmission component. The third transmission component is driven by the second transmission component to rotate when the first transmission component drives the second transmission component to rotate. The sliding component is at least partially slidably disposed inside the third transmission component and coupled to the third transmission component. The sliding component is connected to the plunger. The sliding component is driven by the third transmission component to slide relative to the third transmission component when the third transmission component rotates. The bracket includes a guiding portion. The sliding component passes through the guiding portion. The third transmission component is rotatable relative to the bracket, and the guiding portion is configured to guide the sliding component to slide without a rotation. The guiding portion includes a sliding through hole structure. The sliding component slidably passes through the sliding through hole structure. A cross section of the sliding component matches with a cross section of the sliding through hole structure. The sliding component includes at least one first arc part and at least one first flat part connected to the at least one first arc part. The sliding through hole structure includes at least one second arc part and at least one second flat part connected to the at least one second arc part, and the at least one second arc part and the at least one second flat part are respectively corresponding to the at least one first arc part and the at least one first flat part.

According to an embodiment of the present invention, when the driving component drives the first transmission component to rotate around a first rotating axis, the first transmission component drives the second transmission component to rotate around a second rotating axis so as to drive the third transmission component to rotate around the second rotating axis together with the second transmission component, so that the sliding component is driven to slide relative to the third transmission component along a sliding direction parallel to the second rotating axis without the rotation around the second rotating axis.

According to an embodiment of the present invention, the first transmission component is a worm screw. The second transmission component is a worm gear. The third transmission component is a screw sleeve. The sliding component is a screw rod, and the driving component is an electric motor.

According to an embodiment of the present invention, the driving mechanism further includes a reducer coupled between the driving component and the first transmission component.

According to an embodiment of the present invention, the reducer is a gearbox.

According to an embodiment of the present invention, an internal thread structure is formed on an inner periphery of the third transmission component, and an outer thread structure is formed on the at least one first arc part of the sliding component.

According to an embodiment of the present invention, the bracket further includes a holding portion. The holding portion includes a rotation through hole structure, and the third transmission component rotatably passes through the rotation through hole structure.

According to an embodiment of the present invention, the bracket further includes a supporting portion. The supporting portion includes a platform structure, and the platform structure is configured to support a side of the third transmission component.

According to an embodiment of the present invention, the bracket further includes an accommodating portion. The accommodating portion includes an L-shaped structure, and the L-shaped structure is configured to accommodate the first transmission component.

Furthermore, the claimed auto-injector includes a reservoir, a plunger and one of the aforementioned driving mechanisms.

In summary, in the present invention, the driving mechanism not only has compact structure and high power and high efficiency transmission but also achieves a long sliding distance and a slow sliding speed of the sliding component and prevents any rotation of the sliding component during a sliding movement of the sliding component. Therefore, the present invention can meet requirements of small volume, high driving power, long driving distance, long injecting period and accurate drug dose delivery rate.

These and other objectives of the present invention will no doubt become obvious to those of ordinary skill in the art after reading the following detailed description of the preferred embodiment that is illustrated in the various figures and drawings.

### Brief Description of the Drawings

In the following, the invention is further illustrated by way of example, taking reference to the accompanying drawings. Thereof
FIG. 1 and FIG. 2 are partial diagrams of an auto-injector at different views according to an embodiment of the present invention,
FIG. 3 and FIG. 4 are partial exploded diagrams of the auto-injector at different views according to the embodiment of the present invention,
FIG. 5 and FIG. 6 are partial diagrams of a driving mechanism according to the embodiment of the present invention,
FIG. 7 is a partial sectional diagram of the driving mechanism according to the embodiment of the present invention, and
FIG. 8 and FIG. 9 are diagrams of the auto-injector in different states according to the embodiment of the present invention.

### Detailed Description

In the following detailed description of the preferred embodiments, reference is made to the accompanying drawings which form a part hereof, and in which is shown by way of illustration specific embodiments in which the invention may be practiced. In this regard, directional terminology, such as "top", "bottom", "left", "right", "front", "back", etc., is used with reference to the orientation of the Figure(s) being described. The components of the present invention can be positioned in a number of different orientations. As such, the directional terminology is used for purposes of illustration and is in no way limiting. Accordingly, the drawings and descriptions will be regarded as illustrative in nature and not as restrictive. Also, if not specified, the term "connect" or "couple" is intended to mean either an indirect or direct electrical/mechanical connection. Thus, if a first device is connected or coupled to a second device, that connection may be through a direct electrical/mechanical connection, or through an indirect electrical/mechanical connection via other devices and connections.

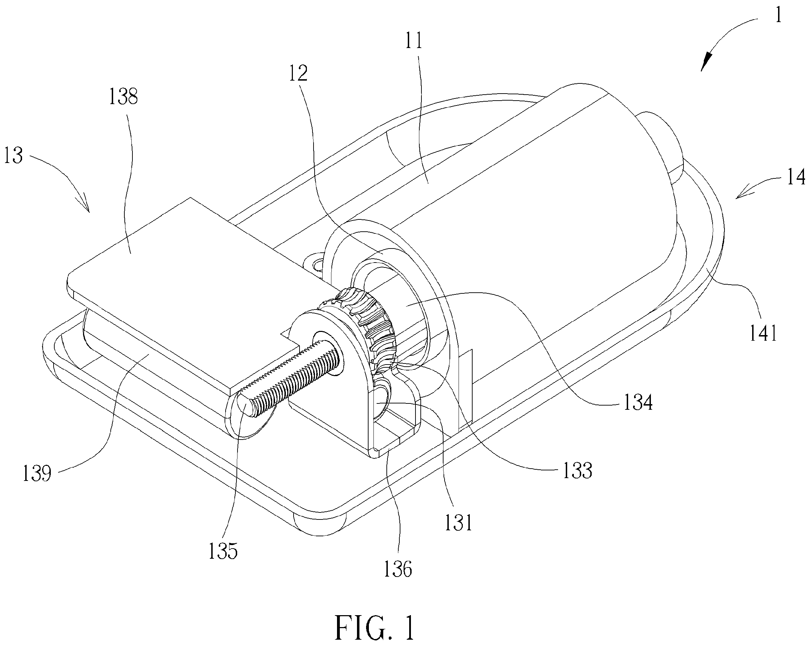

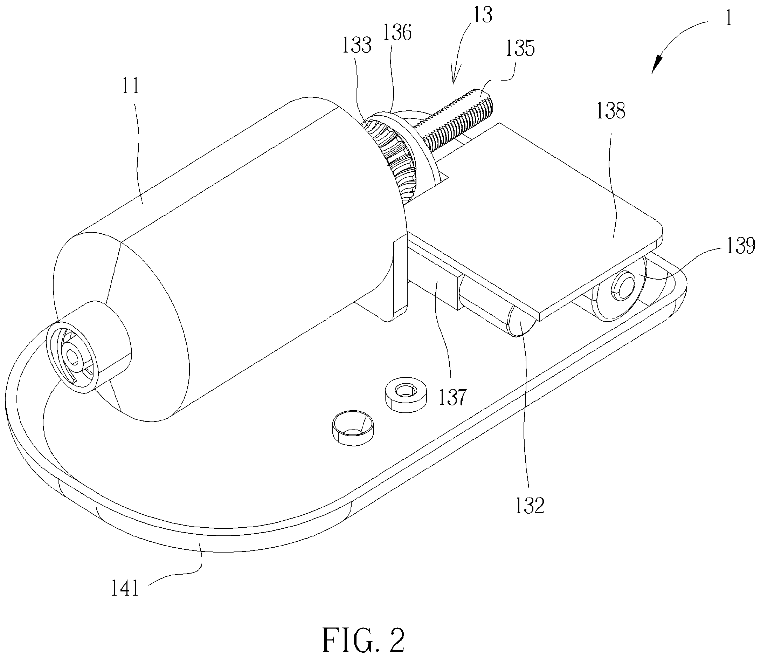

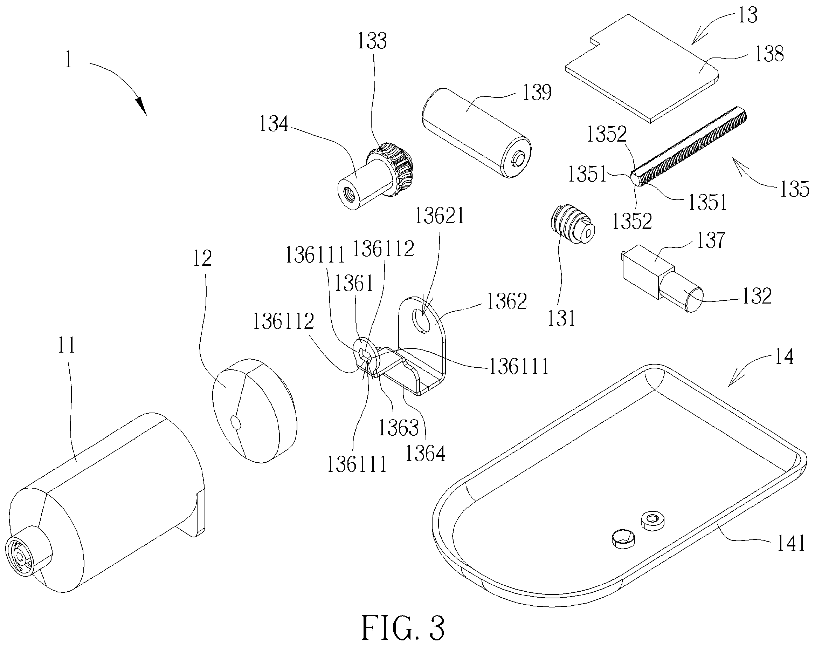

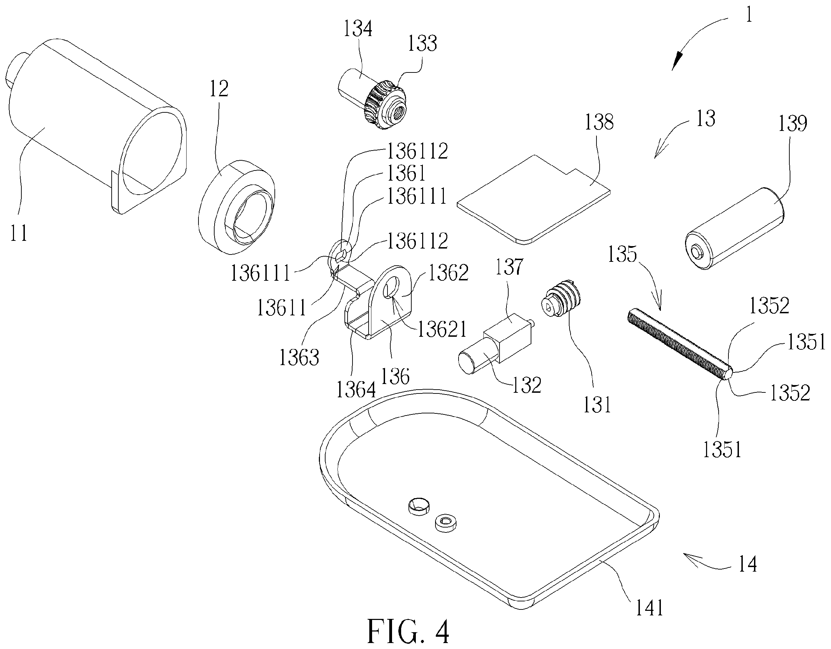

Please refer to FIG. 1 to FIG. 4. FIG. 1 and FIG. 2 are partial diagrams of an auto-injector 1 at different views according to an embodiment of the present invention. FIG. 3 and FIG. 4 are partial exploded diagrams of the auto-injector 1 at different views according to the embodiment of the present invention. As shown in FIG. 1 to FIG. 4, the auto-injector 1 includes a reservoir 11, a plunger 12 and a driving mechanism 13. The plunger 12 is slidably disposed inside the reservoir 11. The driving mechanism 13 is for driving the plunger 12 to slide relative to the reservoir 11.

In this embodiment, the auto-injector 1 further includes a case 14. The case 14 includes a first mounting part 141 and a second mounting part, which is not shown in the figures, detachable installed on the first mounting part 141. The reservoir 11 and the driving mechanism 13 are mounted on the first mounting part 141. The case 14 is configured to conceal the reservoir 11 and the driving mechanism 13 for preventing damage of the reservoir 11 and the driving mechanism 13.

However, the present invention is not limited to this embodiment. For example, in another embodiment, the case can be a one-piece structure with an opening to expose the reservoir and the driving mechanism.

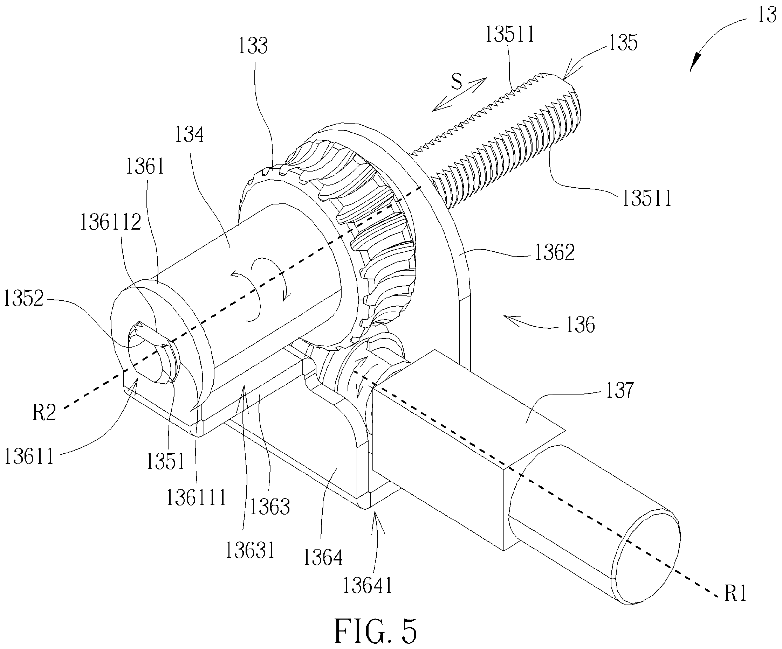

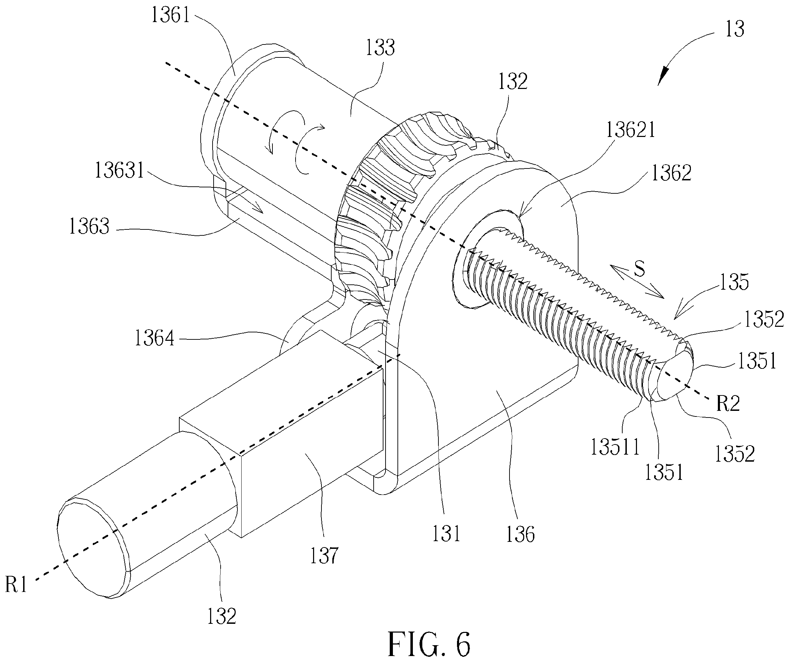

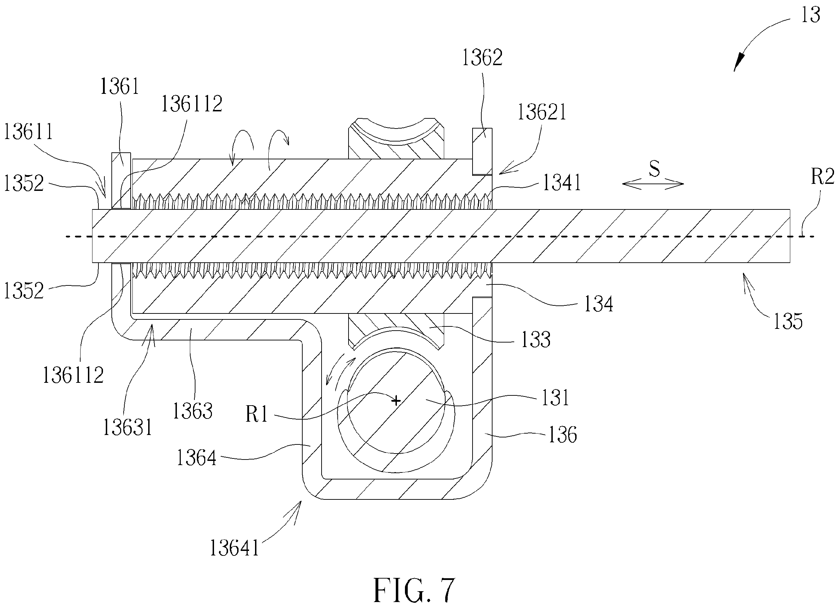

Please refer to FIG. 1 to FIG. 7. FIG. 5 and FIG. 6 are partial diagrams of the driving mechanism 13 according to the embodiment of the present invention. FIG. 7 is a partial sectional diagram of the driving mechanism 13 according to the embodiment of the present invention. As shown in FIG. 1 to FIG. 7, the driving mechanism 13 includes a first transmission component 131, a driving component 132, a second transmission component 133, a third transmission component 134, a sliding component 135 and a bracket 136. The driving component 132 is coupled to the first transmission component 131 and for driving the first transmission component 131 to rotate. The second transmission component 133 is rotatably engaged with the first transmission component 131. The second transmission component 133 is driven by the first transmission component 131 to rotate when the driving component 132 drives the first transmission component 131 to rotate. The third transmission component 134 is fixedly connected to the second transmission component 133, e.g., by a tightly fitting manner or an integrally forming manner. The third transmission component 134 is driven by the second transmission component 133 to rotate when the first transmission component 131 drives the second transmission component 133 to rotate. The sliding component 135 is at least partially slidably disposed inside the third transmission component 134 and coupled to the third transmission component 134. The sliding component 135 is connected to the plunger 12. The sliding component 135 is driven by the third transmission component 134 to slide relative to the third transmission component 134 when the third transmission component 134 rotates. The bracket 136 includes a guiding portion 1361. The sliding component 135 passes through the guiding portion 1361. The third transmission component 134 is rotatable relative to the bracket 136, and the guiding portion 1361 is configured to guide the sliding component 135 to slide without any rotation.

When the driving component 132 drives the first transmission component 131 to rotate around a first rotating axis R1, the first transmission component 131 drives the second transmission component 133 to rotate around a second rotating axis R2 perpendicular to the first rotating axis R1 so as to drive the third transmission component 134 to rotate around the second rotating axis R2 together with the second transmission component 133, so that the sliding component 135 is driven to slide relative to the third transmission component 134 along a sliding direction S parallel to the second rotating axis R2 without any rotation around the second rotating axis R2.

Specifically, the first transmission component 131 can be a worm screw. The second transmission component 133 can be a worm gear. The third transmission component 134 can be a screw sleeve. The sliding component 135 can be a screw rod. The driving component 132 can be an electric motor. However, the present invention is not limited to this embodiment. It depends on practical demands. For example, in another embodiment, the driving component can be a pneumatic motor, and the first transmission component can be a smaller gear wheel. The second transmission component can be a larger gear wheel.

Besides, in this embodiment, as shown in FIG. 1 to FIG. 7, the driving mechanism 13 further includes a reducer 137 coupled between the driving component 132 and the first transmission component 131. An input shaft and an output shaft of the reducer 137 can be respectively connected to the driving component 132 and the first transmission component 131. The reducer 137 can have various reduction ratios to control a rotating speed of the first transmission component 131, so as to control a sliding speed of the sliding component 135.

Specifically, the reducer 137 can be a gearbox. However, the present invention is not limited to this embodiment. It depends on practical demands. For example, in another embodiment, the reducer can be a pulley and belt system. Alternatively, in another embodiment, the reducer can be omitted.

It should be noticed that the aforementioned configuration can not only have small occupied space and achieve high power and high efficiency transmission but also allow a rotating speed of the third transmission component 134 to be adjusted by adjusting a first reduction ratio between the first transmission component 131 and the second transmission component 133 and/or a second reduction ratio between the driving component 132 and the first transmission component, e.g., a gear ratio of the reducer 137.

Furthermore, in this embodiment, as shown in FIG. 1 to FIG. 4, the driving mechanism 13 further includes a control unit 138 and a power source 139. The control unit 138 can be a circuit board for controlling the driving component 132 to actuate or stop a rotating movement of the first transmission component 131 and to control the rotating speed or a rotating direction of the first transmission component 131. The power source 139 can be a battery for providing electricity to the control unit 138 and the driving component 132.

In order to achieve configuration of the guiding portion 1361 to guide the sliding component 135 to slide without any rotation when the third transmission component 134 rotatably drives the sliding component 135 to slide, as shown in FIG. 3 to FIG. 7, the guiding portion 1361 includes a sliding through hole structure 13611. The sliding component 135 slidably passes through the sliding through hole structure 13611. A cross section of the sliding component 135 matches with a cross section of the sliding through hole structure 13611. The sliding component 135 includes two first arc parts 1351 opposite to each other, and two first flat parts 1352 opposite to each other and connected to the two first arc parts 1351. The sliding through hole structure 13611 includes two second arc parts 136111 opposite to each other, and two second flat parts 136112 opposite to each other and connected to the two second arc parts 136111. The two second arc parts 136111 and the two second flat parts 136112 are respectively corresponding to the two first arc parts 1351 and the two first flat parts 1352. An internal thread structure 1341 is formed on an inner periphery of the third transmission component 134, e.g., by plastic injection molding or insert molding, and an outer thread structure 13511 is formed on each of the first arc parts 1351 of the sliding component 135. The aforementioned configuration can ensure no rotation of the sliding component 135 during a sliding movement of the sliding component 135 by a cooperation of the sliding component 135 and the sliding through hole structure 13611, so as to meet a requirement of accurate drug dose delivery rate.

However, the structures of the sliding component and the guiding portion are not limited to this embodiment. For example, in another embodiment, the sliding component can include only one first arc part and one first flat part connected to the first arc part, and the sliding through hole structure can include only one second arc parts and one second flat part connected to the second arc part.

In addition, in order to achieve a stable rotating movement of the third transmission component 134 and make structure of the driving mechanism reasonably compact, as shown in FIG. 3 to FIG. 7, the bracket 136 further includes a holding portion 1362, a supporting portion 1363 and an accommodating portion 1364. The holding portion 1362 is opposite to the guiding portion 1361 and includes a rotation through hole structure 13621. The third transmission component 134 rotatably passes through the rotation through hole structure 1362 and is located between the holding portion 1362 and the guiding portion 1361. The supporting portion 1363 is connected to the guiding portion 1361 and includes a platform structure 13631. The platform structure 13631 can support a lower side of the third transmission component 134 to prevent the third transmission component 134 from accidently falling down. The accommodating portion 1364 is connected to the supporting portion 1363 and the holding portion 1362 and includes an L-shaped structure 13641 for accommodating the first transmission component 131.

However, the present invention is not limited to this embodiment. It depends on practical demands. For example, in another embodiment, the accommodating portion can include a bending structure, a step-shaped structure, or a U-shaped structure. Alternatively, in another embodiment, at least one of the holding portion, the supporting portion and the accommodating portion can be omitted.

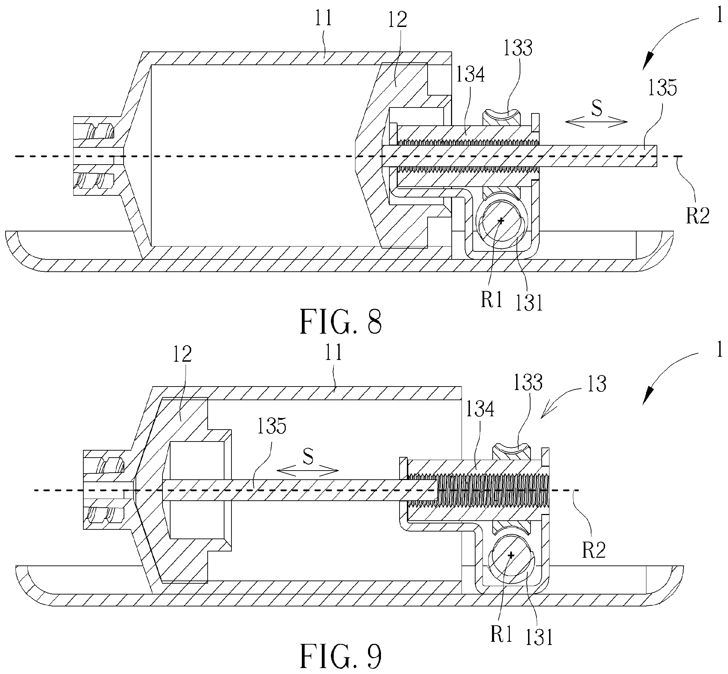

Detailed description for operational principle of the auto-injector 1 is provided as follows. Please further refer to FIG. 5 to FIG. 9. FIG. 8 and FIG. 9 are diagrams of the auto-injector 1 in different states according to the embodiment of the present invention. As shown in FIG. 5 to FIG. 9, after the auto-injector 1 is attached on a patient's body, the driving component 132 can be controlled by the control unit 138 to drive the first transmission component 131 to rotate around the first rotating axis R1 with the reducer 137. When the first transmission component 131 is driven to rotate around the first rotating axis R1, the first transmission component 131 drives the second transmission component 133 to rotate around the second rotating axis R2 so as to drive the third transmission component 134 to rotate around the second rotating axis R2 together with the second transmission component 133, so that the sliding component 135 can be driven to slide relative to the third transmission component 134 along the sliding direction S without any rotation, so as to drive the plunger 12 to slide relative to the reservoir 11 from a position as shown in FIG. 8 to a position as shown in FIG. 9 to stably pump a drug out of the reservoir 11 to complete a drug injection.

In contrast to the prior art, in the present invention, the driving mechanism not only has compact structure and high power and high efficiency transmission but also achieves a long sliding distance and a slow sliding speed of the sliding component and prevents any rotation of the sliding component during a sliding movement of the sliding component. Therefore, the present invention can meet requirements of small volume, high driving power, long driving distance, long injecting period and accurate drug dose delivery rate.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. A driving mechanism (13) for driving a plunger (12) of an auto-injector (1) to slide relative to a reservoir (11) of the auto-injector (1), the driving mechanism (13) comprising:
a first transmission component (131);
a driving component (132) coupled to the first transmission component (131) and for driving the first transmission component (131) to rotate;
a second transmission component (133) rotatably engaged with the first transmission component (131), the second transmission component (133) being driven by the first transmission component (131) to rotate when the driving component (132) drives the first transmission component (131) to rotate;
a third transmission component (134) fixedly connected to the second transmission component (133), the third transmission component (134) being driven by the second transmission component (133) to rotate when the first transmission component (131) drives the second transmission component (133) to rotate;
a sliding component (135) at least partially slidably disposed inside the third transmission component (134) and coupled to the third transmission component (134), the sliding component (135) being connected to the plunger (12), the sliding component (135) being driven by the third transmission component (134) to slide relative to the third transmission component (134) when the third transmission component (134) rotates; and
a bracket (136) comprising a guiding portion (1361), the sliding component (135) passing through the guiding portion (1361), the third transmission component (134) being rotatable relative to the bracket (136), and the guiding portion (1361) being configured to guide the sliding component (135) to slide without a rotation;
**characterized in that** the guiding portion (1361) comprises a sliding through hole structure (13611), the sliding component (135) slidably passes through the sliding through hole structure (13611), a cross section of the sliding component (135) matches with a cross section of the sliding through hole structure (13611), the sliding component (135) comprises at least one first arc part (1351) and at least one first flat part (1352) connected to the at least one first arc part (1351), the sliding through hole structure (13611) comprises at least one second arc part (136111) and at least one second flat part (136112) connected to the at least one second arc part (136111), and the at least one second arc part (136111) and the at least one second flat part (136112) are respectively corresponding to the at least one first arc part (1351) and the at least one first flat part (1352).

2. The driving mechanism (13) of claim 1, **characterized in that** when the driving component (132) drives the first transmission component (131) to rotate around a first rotating axis (R1), the first transmission component (131) drives the second transmission component (133) to rotate around a second rotating axis (R2) so as to drive the third transmission component (134) to rotate around the second rotating axis (R2) together with the second transmission component (133), so that the sliding component (135) is driven to slide relative to the third transmission component (134) along a sliding direction (S) parallel to the second rotating axis (R2) without the rotation around the second rotating axis (R2).

3. The driving mechanism (13) of any one of claims 1 to 2, **characterized in that** the first transmission component (131) is a worm screw, the second transmission component (133) is a worm gear, the third transmission component (134) is a screw sleeve, the sliding component (135) is a screw rod, and the driving component (132) is an electric motor.

4. The driving mechanism (13) of any one of claims 1 to 3, further **characterized by** a reducer (137) coupled between the driving component (132) and the first transmission component (131).

5. The driving mechanism (13) of claim 4, **characterized in that** the reducer (137) is a gearbox.

6. The driving mechanism (13) of claim 1, **characterized in that** an internal thread structure (1341) is formed on an inner periphery of the third transmission component (134), and an outer thread structure (13511) is formed on the at least one first arc part (1351) of the sliding component (135).

7. The driving mechanism (13) of any one of claims 1 to 6, **characterized in that** the bracket (136) further comprises a holding portion (1362), the holding portion (1362) comprises a rotation through hole structure (13621), and the third transmission component (134) rotatably passes through the rotation through hole structure (13621).

8. The driving mechanism (13) of any one of claims 1 to 7, **characterized in that** the bracket (136) further comprises a supporting portion (1363), the supporting portion (1363) comprises a platform structure (13631), and the platform structure (13631) is configured to support a side of the third transmission component (134).

9. The driving mechanism (13) of any one of claims 1 to 8, **characterized in that** the bracket (136) further comprises an accommodating portion (1364), the accommodating portion (1364) comprises an L-shaped structure (13641), and the L-shaped structure (13641) is configured to accommodate the first transmission component (131).

10. An auto-injector (1) comprising a reservoir (11) and a plunger (12) slidably disposed inside the reservoir (11), **characterized in that** the auto-injector (1) further comprises the driving mechanism (13) of any one of claims 1 to 9.

## Patentansprüche

1. Antriebsmechanismus (13) zum Antreiben eines Kolbens (12) eines Autoinjektors (1), damit dieser relativ zu einem Reservoir (11) des Autoinjektors (1) gleitet, worin der Antriebsmechanismus (13) umfasst:
eine erste Übertragungskomponente (131);
eine Antriebskomponente (132), die mit der ersten Übertragungskomponente (131) gekoppelt ist und die erste Übertragungskomponente (131) in Drehung versetzt;
eine zweite Übertragungskomponente (133), die drehbar mit der ersten Übertragungskomponente (131) in Eingriff steht, wobei die zweite Übertragungskomponente (133) von der ersten Übertragungskomponente (131) in Drehung versetzt wird, wenn die Antriebskomponente (132) die erste Übertragungskomponente (131) in Drehung versetzt;
eine dritte Übertragungskomponente (134), die fest mit der zweiten Übertragungskomponente (133) verbunden ist, wobei die dritte Übertragungskomponente (134) von der zweiten Übertragungskomponente (133) in Drehung versetzt wird, wenn die erste Übertragungskomponente (131) die zweite Übertragungskomponente (133) in Drehung versetzt;
eine Gleitkomponente (135), die zumindest teilweise gleitend innerhalb der dritten Übertragungskomponente (134) angeordnet und mit der dritten Übertragungskomponente (134) gekoppelt ist, worin die Gleitkomponente (135) mit dem Kolben (12) verbunden ist, wobei die Gleitkomponente (135) von der dritten Übertragungskomponente (134) angetrieben wird, um relativ zur dritten Übertragungskomponente (134) zu gleiten, wenn sich die dritte Übertragungskomponente (134) dreht; und
eine Halterung (136) mit einem Führungsabschnitt (1361), worin die Gleitkomponente (135) durch den Führungsabschnitt (1361) verläuft, die dritte Übertragungskomponente (134) relativ zur Halterung (136) drehbar ist und der Führungsabschnitt (1361) so ausgebildet ist, dass er die Gleitkomponente (135) zum Gleiten ohne Drehung führt;
**dadurch gekennzeichnet, dass** der Führungsabschnitt (1361) eine Gleitdurchgangslochstruktur (13611) umfasst, worin die Gleitkomponente (135) gleitend durch die Gleitdurchgangslochstruktur (13611) verläuft, worin ein Querschnitt der Gleitkomponente (135) mit einem Querschnitt der Gleitdurchgangslochstruktur (13611) übereinstimmt, die Gleitkomponente (135) mindestens einen ersten Bogenteil (1351) und mindestens einen ersten Flachteil (1352) umfasst, der mit dem mindestens einen ersten Bogenteil (1351) verbunden ist, die Gleitdurchgangslochstruktur (13611) mindestens einen zweiten Bogenteil (136111) und mindestens einen zweiten Flachteil (136112) umfasst, das mit dem mindestens einen zweiten Bogenteil (136111) verbunden ist, und das mindestens eine zweite Bogenteil (136111) und das mindestens eine zweite flache Teil (136112) jeweils dem mindestens einen ersten Bogenteil (1351) und dem mindestens einen ersten flachen Teil (1352) entsprechen.

2. Antriebsmechanismus (13) nach Anspruch 1, **dadurch gekennzeichnet, dass**, wenn die Antriebskomponente (132) die erste Übertragungskomponente (131) antreibt, um sich um eine erste Drehachse (R1) zu drehen, die erste Übertragungskomponente (131) die zweite Übertragungskomponente (133) antreibt, um sich um eine zweite Drehachse (R2) zu drehen , um die dritte Übertragungskomponente (134) dazu anzutreiben, sich zusammen mit der zweiten Übertragungskomponente (133) um die zweite Drehachse (R2) zu drehen, so dass die Gleitkomponente (135) dazu angetrieben wird, relativ zur dritten Übertragungskomponente (134) entlang einer Gleitrichtung (S) parallel zur zweiten Drehachse (R2) zu gleiten, ohne sich um die zweite Drehachse (R2) zu drehen.

3. Antriebsmechanismus (13) nach einem der Ansprüche 1 bis 2, **dadurch gekennzeichnet, dass** die erste Übertragungskomponente (131) eine Schneckenschraube ist, die zweite Übertragungskomponente (133) ein Schneckenrad ist, die dritte Übertragungskomponente (134) eine Schraubhülse ist, die Gleitkomponente (135) eine Gewindestange ist und die Antriebskomponente (132) ein Elektromotor ist.

4. Antriebsmechanismus (13) nach einem der Ansprüche 1 bis 3, ferner **gekennzeichnet durch** eine □ntersetzung (137), die zwischen der Antriebskomponente (132) und der ersten Übertragungskomponente (131) gekoppelt ist.

5. Antriebsmechanismus (13) nach Anspruch 4, **dadurch gekennzeichnet, dass** die □ntersetzung (137) ein Getriebe ist.

6. Antriebsmechanismus (13) nach Anspruch 1, **dadurch gekennzeichnet, dass** eine Innengewindestruktur (1341) an einem Innenumfang der dritten Übertragungskomponente (134) ausgebildet ist und eine Außengewindestruktur (13511) an dem mindestens einen ersten Bogenteil (1351) der Gleitkomponente (135) ausgebildet ist.

7. Antriebsmechanismus (13) nach einem der Ansprüche 1 bis 6, **dadurch gekennzeichnet, dass** die Halterung (136) ferner einen Halteabschnitt (1362) umfasst, worin der Halteabschnitt (1362) eine Drehdurchgangslochstruktur (13621) umfasst und die dritte Übertragungskomponente (134) drehbar durch die Drehdurchgangslochstruktur (13621) hindurchgeht.

8. Antriebsmechanismus (13) nach einem der Ansprüche 1 bis 7, **dadurch gekennzeichnet, dass** die Halterung (136) ferner einen Stützabschnitt (1363) umfasst, worin der Stützabschnitt (1363) eine Plattformstruktur (13631) umfasst und die Plattformstruktur (13631) ausgestaltet ist, eine Seite der dritten Übertragungskomponente (134) zu stützen.

9. Antriebsmechanismus (13) nach einem der Ansprüche 1 bis 8, **dadurch gekennzeichnet, dass** die Halterung (136) ferner einen Aufnahmeabschnitt (1364) umfasst, worin der Aufnahmeabschnitt (1364) eine L-förmige Struktur (13641) umfasst und die L-förmige Struktur (13641) ausgestaltet ist, die erste Übertragungskomponente (131) aufzunehmen.

10. Autoinjektor (1) mit einem Reservoir (11) und einem Kolben (12), der verschiebbar innerhalb des Reservoirs (11) angeordnet ist, **dadurch gekennzeichnet, dass** der Autoinjektor (1) ferner den Antriebsmechanismus (13) gemäß einem der Ansprüche 1 bis 9 umfasst.

## Revendications

1. Mécanisme d'entraînement (13) destiné à entraîner un piston (12) d'un auto-injecteur (1) afin qu'il coulisse par rapport à un réservoir (11) de l'auto-injecteur (1), le mécanisme d'entraînement (13) comprenant:
un premier composant de transmission (131);
un composant d'entraînement (132) couplé au premier composant de transmission (131) et destiné à entraîner le premier composant de transmission (131) en rotation;
un deuxième composant de transmission (133) engagé de manière rotative avec le premier composant de transmission (131), le deuxième composant de transmission (133) étant entraîné par le premier composant de transmission (131) pour tourner lorsque le composant d'entraînement (132) entraîne le premier composant de transmission (131) pour tourner;
un troisième composant de transmission (134) relié de manière fixe au deuxième composant de transmission (133), le troisième composant de transmission (134) étant entraîné en rotation par le deuxième composant de transmission (133) lorsque le premier composant de transmission (131) entraîne le deuxième composant de transmission (133) en rotation;
un composant coulissant (135) disposé au moins partiellement de manière coulissante à l'intérieur du troisième composant de transmission (134) et couplé au troisième composant de transmission (134), le composant coulissant (135) étant relié au piston (12), le composant coulissant (135) étant entraîné par le troisième composant de transmission (134) pour coulisser par rapport au troisième composant de transmission (134) lorsque le troisième composant de transmission (134) tourne; et
un support (136) comprenant une partie de guidage (1361), le composant coulissant (135) passant à travers la partie de guidage (1361), le troisième composant de transmission (134) pouvant tourner par rapport au support (136), et la partie de guidage (1361) étant configurée pour guider le composant coulissant (135) afin qu'il coulisse sans rotation;
**caractérisé en ce que** la partie de guidage (1361) comprend une structure à trou traversant coulissant (13611), le composant coulissant (135) passe de manière coulissante à travers la structure à trou traversant coulissant (13611), une section transversale du composant coulissant (135) correspond à une section transversale de la structure à trou traversant coulissant (13611), le composant coulissant (135) comprend au moins une première partie arquée (1351) et au moins une première partie plate (1352) reliée à la au moins une première partie arquée (1351), la structure à trou traversant coulissant (13611) comprend au moins une deuxième partie arquée (136111) et au moins une deuxième partie plate (136112) reliée à la au moins une deuxième partie arquée (136111), et la au moins une deuxième partie arquée (136111) et la au moins une deuxième partie plate (136112) correspondent respectivement à la au moins une première partie arquée (1351) et à la au moins une première partie plate (1352).

2. Mécanisme d'entraînement (13) selon la revendication 1, **caractérisé en ce que** lorsque le composant d'entraînement (132) entraîne le premier composant de transmission (131) à tourner autour d'un premier axe de rotation (R1), le premier composant de transmission (131) entraîne le deuxième composant de transmission (133) à tourner autour d'un deuxième axe de rotation (R2) afin d'entraîner le troisième composant de transmission (134) à tourner autour du deuxième axe de rotation (R2) avec le deuxième composant de transmission (133), de sorte que le composant coulissant (135) est entraîné à coulisser par rapport au troisième composant de transmission (134) le long d'une direction de coulissement (S) parallèle au deuxième axe de rotation (R2) sans rotation autour du deuxième axe de rotation (R2).

3. Mécanisme d'entraînement (13) de l'une quelconque des revendications 1 à 2, **caractérisé en ce que** le premier composant de transmission (131) est une vis sans fin, le deuxième composant de transmission (133) est un engrenage à vis sans fin, le troisième composant de transmission (134) est un manchon à vis, le composant coulissant (135) est une tige à vis et le composant d'entraînement (132) est un moteur électrique.

4. Mécanisme d'entraînement (13) selon l'une quelconque des revendications 1 à 3, **caractérisé en outre par** un réducteur (137) couplé entre le composant d'entraînement (132) et le premier composant de transmission (131).

5. Mécanisme d'entraînement (13) selon la revendication 4, **caractérisé en ce que** le réducteur (137) est une boîte de vitesses.

6. Mécanisme d'entraînement (13) selon la revendication 1, **caractérisé en ce qu'**une structure filetée interne (1341) est formée sur une périphérie interne du troisième composant de transmission (134), et une structure filetée externe (13511) est formée sur au moins une première partie arquée (1351) du composant coulissant (135).

7. Mécanisme d'entraînement (13) de l'une quelconque des revendications 1 à 6, **caractérisé en ce que** le support (136) comprend en outre une partie de maintien (1362), la partie de maintien (1362) comprend une structure à trou traversant rotatif (13621), et le troisième composant de transmission (134) passe de manière rotative à travers la structure à trou traversant rotatif (13621).

8. Mécanisme d'entraînement (13) selon l'une quelconque des revendications 1 à 7, **caractérisé en ce que** le support (136) comprend en outre une partie de support (1363), la partie de support (1363) comprend une structure de plate-forme (13631), et la structure de plate-forme (13631) est configurée pour supporter un côté du troisième composant de transmission (134).

9. Mécanisme d'entraînement (13) de l'une quelconque des revendications 1 à 8, **caractérisé en ce que** le support (136) comprend en outre une partie accommodante (1364), la partie accommodante (1364) comprend une structure en forme de L (13641), et la structure en forme de L (13641) est configurée pour accommoder le premier composant de transmission (131).

10. Auto-injecteur (1) comprenant un réservoir (11) et un piston (12) disposé de manière coulissante à l'intérieur du réservoir (11), **caractérisé en ce que** l'auto-injecteur (1) comprend en outre le mécanisme d'entraînement (13) de l'une quelconque des revendications 1 à 9.
